# EUROPÄISCHE PATENTSCHRIFT

(11) **EP 1 660 668 B1**
(45) Veröffentlichungstag und Bekanntmachung des Hinweises auf die Patenterteilung: **25.07.2007**
(21) Anmeldenummer: 04764758.1
(22) Anmeldetag: 02.09.2004
(51) Int. Cl.: C12P 7/00

(54) **VERFAHREN ZUR ENZYMATISCHEN HERSTELLUNG VON MONO- UND DIACYLGLYCERID-HALTIGEN EMULGATOREN**
METHOD FOR THE ENZYMATIC PRODUCTION OF EMULSIFIERS CONTAINING MONO- AND DIACYLGLYCERIDES
PROCEDE POUR LA PRODUCTION ENZYMATIQUE D'EMULSIFIANTS CONTENANT DES MONOACYLGLYCERIDES ET DES DIACYLGLYCERIDES

(30) Priorität: 04.09.2003 DE 10340739
(43) Veröffentlichungstag der Anmeldung: 31.05.2006
(73) Patentinhaber: Cargill, Incorporated, Wayzata MN 55391-1063 (US)
(72) Erfinder: VOLLAND, Michael, 85757 Karlsfeld (DE); LÖTZBEYER, Thomas, 85386 Eching (DE); SIEBER, Volker, 85405 Nandlstadt (DE); WITTMANN, Eva, 83301 Traunreut (DE)
(74) Vertreter: Dey, Michael
(86) Internationale Anmeldenummer: PCT/EP2004/009800
(87) Internationale Veröffentlichungsnummer: WO 2005/024036

(56) Entgegenhaltungen:
- DE-C1- 19 623 735

## Beschreibung

Gegenstand der vorliegenden Erfindung ist ein Verfahren zur enzymatischen Herstellung Mono- und Diacylglycerid-haltiger Emulgatoren und deren Verwendung.

Lecithine und Lysolecithine werden in verschiedenen Anwendungen als Emulgatoren verwendet. Man findet sie in Lebensmitteln, Kosmetika, Nahrungsergänzungsmitteln und chemischen Formulierungen.
Sie werden im Allgemeinen als Nebenprodukte der Ölgewinnung z. B. aus Sojabohnen oder Raps gewonnen oder direkt aus dem entsprechenden Rohstoff, wie z.B. Eigelb extrahiert. Lecithine und Lysolecithine stellen meist eine komplexe Mischung aus Phospholipiden (1, 2-Diacylglycerinphosphat), Lysophospholipiden (1- oder 2-Monoacylglycerinphosphat als Hydrolysederivat von Phospholipiden), Glycolipiden und Triacylglyceriden dar. Je nach Verarbeitungsprozess können die Gehalte der einzelnen Fraktionen im Endprodukt stark schwanken.

Rohlecithin stellt ein nicht standardisiertes Lecithin dar, wie es z.B. in der Ölmühle bei der Raffination von Pflanzenöl als Nebenprodukt anfällt. Der Ausdruck Öl beinhaltet raffiniertes oder unraffiniertes Pflanzenöl, teilweise oder vollständig gehärtetes (hydriertes) Pflanzenöl oder tierisches Fett, deren aller Hauptkomponente Triacylglyceride sind.

Mono-, Di- und Triacylglyceride weisen ein, zwei oder drei Acylgruppen auf, die einer langkettigen gesättigten oder ein- oder mehrfach ungesättigten Fettsäure entstammen. Bevorzugt weisen die Acylgruppen 6 bis 35 C-Atome, insbesondere 10 bis 30 C-Atome, und noch mehr bevorzugt 12 bis 26 C-Atome auf. Monoacylglyceride können 1- oder 2-Monoacylglyceride sein, Diglyceride können 1,2- oder 1,3-Diacylglyceride darstellen.

Höhere Gehalte an Lysolecithinen können mit Hilfe einer enzymatischen Hydrolyse der entsprechenden Phospholipide durch Abspaltung eines oder zweier Fettsäurereste zu den entsprechenden Lysophospholipiden gewonnen werden.

Unter "Lysolecithin" soll im vorliegenden Zusammenhang, wie auch im kommerziellen und lebensmittelrechtlichen Sinn eine Mischung aus enzymatisch teilhydrolysierten polaren Lipiden und neutralen Lipiden verstanden werden, die einen Anteil Acetonunlösliches von mindestens 56% aufweisen. Acetonunlösliches ist dabei einer der wichtigsten Analysenparameter, der zur lebensmittelrechtlichen Beurteilung von Lecithin herangezogen wird und der den Anteil der polaren Lipide im Lecithin, die in Aceton nicht löslich sind, beschreibt.

Die Hydrolyse von Lipiden und Phospholipiden mit Lipasen und Phospholipasen ist gut beschrieben (Adlercreutz, Patrick, 1994: "Enzymecatalyzed lipid modification"; Biotechnology & Genetic Engineering Reviews 12, 231-254).

Darüber hinaus beschäftigt sich eine Vielzahl von Patentdokumenten ebenfalls mit der enzymatischen Hydrolyse von Phospholipiden zu Lysolecithinen.

EP-A 260573 beschreibt die Herstellung von Lysolecithin, indem ein Lecithin mit 5-30 Gew.-% Wasser versetzt und in Gegenwart eines Calciumsalzes sowie eines Lecithin hydrolysierenden Enzyms zu Lysolecithin umgesetzt wird.

In WO 91/03565 wird die Hydrolyse von isolierten Phospholipiden in organischen Lösemitteln mit Hilfe einer immobilisierten Lipase gelehrt, während EP-A 870840 die Hydrolyse einer wässrigen Lecithinlösung mit Phospholipasen beschreibt, bei der die entstehenden Lysophospholipide durch Lösemittelextraktion (Aceton) von Begleitstoffen befreit werden.

Ein weiteres Verfahren zur Herstellung eines Lysolecithins ist in JP 10042884 beschrieben, wo das gewünschte Produkt durch Hydrolyse von Lecithin mittels Phospholipase A2 in einem Lösemittel/Wasser Gemisch hergestellt wird.

WO 97/28270 lehrt ein Verfahren zur Herstellung von Lysophosphatidylcholin aus dem Substrat Phosphatidylcholin mit Hilfe einer Phospholipase A2. Die Reaktion wird in einer Dispersion des Substrats mit einem weiteren Agens aus der Gruppe der Monoacylglyceride, Diacylglyceride, Polyglycerol-, Saccharose- und Sorbitan-Fettsäureester sowie Glycerin in Gegenwart von Wasser durchgeführt.

Neben den Lecithinen und Lysolecithinen wirken auch Mono- und Diacylglyceride als Stabilisatoren für Emulsionen. Sie werden meist durch alkalische Verseifung von Triacylglyceriden bei hohen Temperaturen gewonnen. Sie müssen allerdings im Anschluss an diesen Hydrolyseschritt kostenintensiv aufgereinigt werden.

Eine alternative Methode zur Herstellung von Monoacylglyceriden beschreibt WO 02/11543. Mit Hilfe einer Lipase werden aus dem entsprechenden Triacylglycerid über 40 % als Monoacylglyceride in einem Polyol/Wasser-Gemisch erhalten. Lecithin wie auch Fette werden hierbei ausschließlich zur Erweiterung des Substratspektrums zugesetzt.

In der Regel werden die Mono- und Diacylglyceride zur Stabilisierung von Emulsionen in Kombination mit Lysolecithinen im Backwaren- und Margarinebereich eingesetzt. Dazu werden bisher die Einzelkomponenten meist getrennt hergestellt, aufgereinigt und in der gewünschten Konzentration gemischt.

WO 00/52190 beschreibt ein Verfahren zur Herstellung einer Mischung von Lysolipiden, Lysophospholipiden, Monoacylglyceriden und Diacylglyceriden durch Reaktion von Lecithin in einem Wasser/Polyol-Gemisch in Gegenwart einer Kombination aus Lipase und Phospholipase. Der Schwerpunkt liegt hierbei in der möglichst vollständigen Umsetzung der Phospholipide bei einer Umsetzungsrate von 80 % bezogen auf das Verhältnis von Lysophosphatidylcholin/Phosphatidylcholin. Durch den hohen Wassergehalt der Reaktionslösung von ca. 60 % ist die Gewinnung des Endproduktes allerdings mit einem hohen Energie- und Kostenaufwand verbunden. Eine definierte Einstellung des Lysolecithin/Mono- und Diacylglycerid-Verhältnisses ist mit diesem Verfahren allerdings nicht möglich, da der Lysophospholipid-Anteil immer überwiegt und der Monoacylglycerid-Anteil durch die Zugabe des Glycerins immer wesentlich höher ausfällt als der Diacylglycerid-Anteil.

Allerdings kann das Verhältnis zwischen Mono- und Diacylglyceriden sowie Lysophospholipiden im zu stabilisierenden Produkt in Abhängigkeit von der jeweiligen Anwendung stark variieren. So werden bspw. zur Stabilisierung von Margarine Mono-/ Diacylglyceride und Lysolecithin im Verhältnis 1,7:1 zugegeben, wogegen im Bereich der Backwaren der Anteil des Lysolecithins überwiegt und die entsprechende Mischung im Verhältnis von 0,4:1 vorliegt.

Ausgehend von diesem Kenntnisstand hat sich für die vorliegende Erfindung die Aufgabe gestellt, ein Verfahren zur enzymatischen Herstellung von Mono- und Diacylglycerid-haltigen Emulgatoren bereitzustellen, das ohne die sonst übliche Zugabe von Calcium, Polyolen oder organischen Lösemitteln eine kostengünstige Herstellung von Gemischen aus Mono- und Diacylglyceriden sowie Lysophospholipiden erlaubt und das darüber hinaus eine flexible Einstellung des Verhältnisses zwischen Mono- und Diacylglyceriden sowie Lysophospholipiden durch Variation der Zusammensetzung des Reaktionsgemisches oder der Reaktionsbedingungen ermöglicht.

Gelöst wurde diese Aufgabe mit einem entsprechenden Verfahren, bei dem
a) eine Mischung aus einer Phospholipid- und einer Triacylglycerid-Komponente vorgelegt wird,
b) zu der Mischung aus Verfahrensschritt a) eine solche Menge einer (Phospho-)Lipase-haltigen wässrigen Lösung gegeben wird, dass der Wassergehalt der Mischung zwischen 3 und 15 Gew.-% und insbesondere zwischen 5 und 12 Gew.-% beträgt, anschließend
c) die aus Verfahrensschritt b) erhaltene Mischung bei Temperaturen zwischen 20° und 80°C über einen Zeitraum von mindestens 2 Stunden zur Reaktion gebracht wird, und abschließend
d) die Mischung nach dem Reaktionsende getrocknet wird.

Überraschend hat sich bei der erfindungsgemäßen gleichzeitigen Umsetzung von Phospholipiden (Lecithin) und Triacylglyceriden (Öl) zu Lysophospolipiden und Mono- sowie Diacylglyceriden herausgestellt, dass durch die Wahl der Parameter Temperatur, Inkubationszeit, Verhältnis zwischen Lecithin und Öl, Lecithin und Wasser bzw. Öl und Wasser oder/und der Menge an zugesetztem Enzym die Zusammensetzung des Reaktionsproduktes exakt eingestellt werden kann. So ist es z.B. über das Verhältnis von Lecithin zu Öl, das sich über den Anteil an Acetonunlöslichem definieren lässt, möglich, bei einer Hydrolyse mit Lipase und einem bestimmten Wasseranteil, den Anteil von Monoacylglyceriden und von 1,2-Diacylglyceriden am Gesamt-Mono-/Diacylglycerid-Anteil bei vergleichsweise konstantem Anteil von 1,3-Diacylglyceriden gezielt einzustellen. Alternativ kann aber auch über die Inkubationszeit bspw. bei einer Temperatur von 60°C mit 0,05 Gew.-% Lipase in einem Gemisch Rohlecithin/ Öl, mit einem Anteil an Acetonunlöslichem von 50%, mit einem Wasseranteil von 10%, der Anteil von Monoacylglyceriden und von 1,3-Diacylglyceriden am Gesamt-Mono-/Diacylglycerid-Anteil variiert werden, wobei der Anteil an 1,2-Diacylglyceriden am Gesamt-Mono-/Diacylglycerid-Gehalt vergleichsweise konstant bleibt. Diese Vorteile waren unter Berücksichtigung der Einfachheit der Verfahrensführung in diesem Ausmaß nicht zu erwarten.

Als Phospholipidkomponente hat sich erfindungsgemäß ein Lecithin und vorzugsweise Rohlecithin als besonders geeignet gezeigt, wobei ein Soja-Rohlecithin als besonders bevorzugt anzusehen ist.

Pflanzliche und/oder tierische Öle, vorzugsweise in raffinierter und/oder mindestens teilweise gehärteter Form sind im Rahmen der vorliegenden Erfindung besonders geeignete Vertreter der Triacylglycerid-Komponente, wie sie im Verfahrensschritt a) vorgelegt wird.

Hinsichtlich der Verfahrensführung kann in einer speziellen Variante im Verfahrensschritt a) eine Mischung vorgelegt werden, die einen Phospholipidkomponenten-Anteil zwischen 10 und 80 Gew.-% und/oder einen Triacylglyceridkomponenten-Anteil zwischen 20 und 90 Gew.-% aufweist. Das Gew.-Verhältnis zwischen Phospolipidkomponenten-Anteil und Triacylglyceridkomponenten-Anteil beträgt vorteilhafterweise 1 : 0,25 bis 1 : 4.

Generell ist der Verfahrensschritt a) keiner besonderen Einschränkung unterworfen, allerdings kann es unter gewissen Bedingungen vorteilhaft sein, dass die Mischung im Verfahrensschritt a) auf eine Temperatur zwischen 35° und 60°C gebracht wird, was die vorliegende Erfindung ebenfalls berücksichtigt.

Hinsichtlich des Verfahrensschrittes b) ist es als bevorzugt anzusehen, wenn eine Lipase und/oder Phospholipase mikrobiellen Ursprungs, vorzugsweise aus Candida und/oder Aspergillus eingesetzt wird. Besonders geeignete Stämme sind dabei *Aspergillus niger* und *Candida cylindracea,* wobei natürlich auch jede andere geeignete Enzymquelle gewählt werden kann.

Wie bereits dargelegt, liegt ein großer Vorteil des vorliegenden Verfahrens darin, dass das Verhältnis von Mono- und Diacylglyceriden im emulgierend wirkenden Produkt gezielt einstellbar ist. Die Erfindung sieht deshalb auch vor, dass im Verfahrensschritt b) eine (Phospho-)Lipase-Menge von 0,05 bis 10,0 mg pro ml-Reaktionsmischung und insbesondere 0,1 bis 5 mg pro ml-Reaktionsmischung verwendet wird. Die jeweils gewünschte Enzymmenge kann dabei selbstverständlich auch in Abhängigkeit von der Enzymaktivität eingesetzt werden, weshalb eine entsprechende Menge zwischen 0,1 und 120 u pro ml Reaktionsgemisch empfehlenswert ist. Allgemein kann das Verhältnis von Lipase zu Phospholipase in der eingesetzten Enzym-Komponente in einem breiten Bereich variiert werden, wodurch gezielt Einfluss auf die Zusammensetzung des erhaltenen Produktes (des Emulgators) genommen werden kann.

Als bevorzugt ist im Hinblick auf den Verfahrensschritt c) eine Temperatur anzusehen, die zwischen 40 und 50°C liegt. Die Reaktionsdauer im Verfahrensschritt c) sollte vorteilhafterweise zwischen 5 und 20 Stunden liegen und besonders bevorzugt zwischen 8 und 12 Stunden.

Der abschließende Trocknungsschritt d) ist im vorliegenden Verfahren wieder überhaupt nicht limitierend und kann mit jedem üblichen Verfahren durchgeführt werden. Es sollte allerdings auf schonende Bedingungen geachtet werden, weshalb Temperaturen besonders geeignet sind, die zwischen 60° und 80°C liegen. Die Durchführung der Trocknung im Vakuum ist besonders empfehlenswert.

Typischerweise wird das erfindungsgemäße Verfahren durchgeführt, indem Lecithin mit Öl in einem spezifischen Verhältnis vereint, auf eine Temperatur zwischen 35°-60°C temperiert und durch Rühren gemischt wird. Eine mögliche Quelle für das Lecithin ist dabei Rohlecithin aus Soja, wobei statt Rohlecithin auch ein standardisiertes, entöltes oder fraktioniertes Lecithin verwendet werden kann. Als Öl kommen pflanzliche Öle, wie z.B. Sojaöl, oder ein hydriertes pflanzliches Öl, wie z.B. Palmfett, oder aber auch tierische Öle und Fette in Frage. Die Enzym-Komponente wird als Lipase in einer wässrigen Lösung zu dem Lecithin/Öl-Gemisch gegeben, wobei der Gesamtanteil an Wasser bevorzugt im Bereich 6 bis 12 Gew.-% liegt. Mögliche Quellen für Lipase-Enzyme sind dabei insbesondere *Candida cylindracea* (vgl. Biocatalysts, Pontypridd, Wales) oder *Aspergillus niger* (vgl. Amano, Nagoya, Japan). Das Reaktionsgemisch wird anschließend bei einer bestimmten Temperatur und für eine bestimmte Zeit gerührt, wobei die Temperatur z.B. im Bereich von 50°C liegen kann. Als Inkubationszeit ist bevorzugt eine Spanne zwischen 2 und 15 Stunden vorgesehen. Nach Ablauf dieser Reaktion wird das Gemisch unter Vakuum bei einer Temperatur von ca. 70°C getrocknet, und das Produkt ggf. noch kristallisiert.

Als bevorzugtes Produkt wird von der vorliegenden Erfindung eine Mischung beansprucht, die aus Lysolecithin, Mono- und Diacylglyceriden besteht, wobei die bevorzugten Anteile bzgl. Lysolecithin zwischen 3,0 und 55 Gew.-% und bzgl. der Monoacylglyceride zwischen 2,0 bis 20 Gew.-% liegen sollten. Im Hinblick auf den Diacylglycerid-Anteil werden Mengen empfohlen, die zwischen 6,0 und 40 Gew.-% liegen.

Alternativ sieht die Erfindung als Produkt aus dem beanspruchten Verfahren ein Gemisch vor, bei dem das Verhältnis Phospholipid-Komponente zu der kombinierten Mono- und Diacylglycerid-Komponente 1 : 0,25 bis 4,0 beträgt.

Die Reaktionsprodukte können nach der Trocknung direkt als Emulgatoren in Standardverfahren zur Herstellung von Margarine oder Backmischungen verwendet werden. Dabei ist keine Zugabe weiterer Komponenten wie z.B. zusätzlicher Mono- oder Diacylglyceride notwendig.

Neben dem Verfahren selbst und den damit hergestellten Gemischen beansprucht die vorliegende Erfindung gemäss Anspruch 14 auch die Verwendung der mit dem Verfahren herstellbaren Gemische zur Herstellung von Emulsionen und Cremes im Lebensmittelbereich, insbesondere in Form von Eiscremes, Margarinen und Backwaren, und im Kosmetikbereich.

Im Rahmen dieser Erfindung wurde schließlich gefunden, dass die Produkte und damit der Emulgator im Gegensatz zu bisher erhältlichen Emulgatoren, die durch Mischung der reinen Lysolecithin- und Mono-/Diacylglycerid-Bestandteile hergestellt werden, auch in flüssiger Form vorliegen können und die deshalb ohne weitere Bearbeitung leicht verwendbar sind, ohne dabei Qualitätseinbußen zu erleiden.

Mit dem beanspruchten Verfahren gelingt durch die gleichzeitige enzymatische Umsetzung von Phospholipiden (Lecithin) und Triacylglyceriden (Öl) zu Lysophospholipiden und Mono-/Diacylglyceriden auf einfache Weise die Herstellung von Emulgatoren. Die Umsetzung von Lecithin in Lysolecithin erfolgt dabei über eine enzymatische Hydrolyse, ohne dass hierfür organische Lösemittel, Polyole und/oder Calcium zugesetzt werden müssen, oder dass ein Wasseranteil > 15 Gew.-% verwendet werden muss. Darüber hinaus ist im Rahmen des erfindungsgemäßen Verfahrens eine exakte Einstellung der Anteile einzelner Lipidkomponenten im Produkt zielgenau möglich, indem ausgewählte Eingangsparameter variiert werden. Schließlich ist ein weiterer Vorteil der Erfindung darin zu sehen, dass die so erhaltenen Emulgatoren auch direkt in flüssiger Form gewonnen werden können.

Bei dem beschriebenen Verfahren zur enzymatischen Herstellung von Mono- und Diacylglycerid-haltigen Emulgatoren wird in einem ersten Verfahrensschritt a) eine Mischung aus einer Phospholipid- und einer Triacylglycerid-Komponente vorgelegt, anschließend im Verfahrensschritt b) zu der so erhaltenen Mischung eine solche Menge einer (Phospho-) Lipasehaltigen wässrigen Lösung gegeben, dass der Wassergehalt der Mischung zwischen 3 und 15 Gew.-% beträgt. Anschließend wird im Verfahrensschritt c) die aus dem vorhergehenden Schritt erhaltene Mischung bei Temperaturen zwischen 20° und 80°C über einen Zeitraum von mindestens zwei Stunden zur Reaktion gebracht und abschließend wird diese Mischung getrocknet. Als besonders geeignete Komponenten haben sich Soja-Rohlecithin und pflanzliche und/oder tierische Öle gezeigt, die gemeinsam mit einer Mischung aus Lipase und/oder Phospholipase als Enzym-Komponente umgesetzt werden. Mit diesem Verfahren, das ohne die sonst üblichen organische Lösemittel, Polyole oder ionischen Zusätze sowie mit geringem Wasseranteil auskommt, werden Emulgatoren erhalten, deren Zusammensetzung aus Lysophospholipiden und Mono-/Diacylglyceriden gezielt eingestellt werden können. Ihren Einsatz finden diese Emulgatoren, die auch in flüssiger Form erhältlich sind, im Lebensmittel- und Kosmetikbereich.

Die nachfolgenden Beispiele veranschaulichen die genannten Vorteile des beanspruchten Verfahrens zur enzymatischen Herstellung von Emulgatoren.

### Beispiele

Das in den nachfolgenden Beispielen verwendete Rohlecithin stammte von Honeymead, USA; beim Sojaöl handelte es sich um freierwerbbare, handelsübliche Waren.
Die Zusammensetzung der jeweils erhaltenen Produkts wurde mithilfe der HPLC, HPTLC und mit gravimetrischen Methoden analysiert.
Für die HPLC wurde als stationäre Phase eine Kieselgel Si60 Säule verwendet. Die mobile Phase setzte sich aus n-Hexan / 2-Propanol / Wasser (0,25 / 4 / 1 [v/v/v]) zusammen, die mit einer Flussrate von 1,2 mL / min über die Säule gegeben wurde. Anhand der unterschiedlichen Retentionszeiten konnte der Gehalt an Phosphatidylcholin und Lysophosphatidylcholin quantifiziert werden. Die Proben wurden zuvor in n-Hexan / 2-Propanol (2 / 1 [v/v]) gelöst.
Die HPTLC wurde mit Kieselgel Si60, aufgebracht auf eine Glasplatte (Format 20x10 cm), als stationäre Phase durchgeführt. Als mobile Phase diente Diethylether / Petroleumbenzin 1 Eisessig im Verhältnis 40 / 59 / 1 [v/v/v]. Nach dem Analysenlauf und Trocknen der Platte wurden die erhaltenen Banden mit einer Kupfer-(II)-sulfat Lösung (10 Gew.-% Kupfersulfat gelöst in 8%-iger Phosphorsäure) derivatisiert und der Gehalt an Monoacylglyceriden sowie an 1,2- und 1,3-Diacylglyceriden quantifiziert. Die Proben wurden zuvor in Chloroform gelöst.
Die gravimetrische Methode wurde zur Bestimmung des Acetonunlöslichen eingesetzt. Hierbei wurde die Probe mit Aceton versetzt, der erhaltene Rückstand abgetrennt, getrocknet und gewogen.

### Beispiel 1

45 g Rohlecithin wurden mit 55 g Sojaöl gemischt und bei einer Temperatur von 47,5°C gerührt. 0,13 g Lipase aus *Candida cylindracea* (von Biocatalysts, UK) wurden in 9 g Wasser gelöst und anschließend unter Rühren zu dem Lecithin/Öl-Gemisch gegeben. Diese Mischung wurde bei 47,5°C gerührt und nach einer Reaktionszeit von 8,5 h unter einem Vakuum von 0,1 bar und bei einer Temperatur von 80°C getrocknet.
Ergebnis: Das erhaltene Produkt enthielt 28 Gew.-% Acetonunlösliches, was einem Lecithinanteil von 43% entspricht, und ferner 2 Gew.-% Lysophosphatidylcholin, 4,5 Gew.-% Monoacylglyceride und 11 Gew.-% Diacylglyceride. Das Verhältnis Lecithin zu Mono-/Diacylglyceride betrug 1 : 0,34.

### Beispiel 2

135g Rohlecithin wurden mit 165g Sojaöl und 27g Wasser gemischt und bei einer Temperatur von 47,5°C gerührt. 0,15g Lipase aus *Candida cylindracea* wurden unter Rühren zu dem Lecithin/Öl-Gemisch gegeben. Diese Mischung wurde bei 47,5°C gerührt und nach einer Reaktionszeit von 8,5h unter einem Vakuum von 0,1 bar und bei einer Temperatur von 80°C getrocknet.
Ergebnis: Das erhaltene Produkt enthielt 27 Gew.-% Acetonunlösliches, was einem Lecithinanteil von 42% entspricht, und ferner 2,5 Gew.-% Lysophosphatidylcholin, 5 Gew.-% Monoacylglyceride und 10 Gew.-% Diacylglyceride. Das Verhältnis Lecithin zu Mono-/Diacylglyceride betrug 1 : 0,36.

### Beispiel 3

10g Rohlecithin wurden mit 90g Sojaöl gemischt und bei einer Temperatur von 35°C gerührt. 0,1g Lipase aus *Candida cylindracea* wurde in 12g Wasser gelöst und anschließend unter Rühren zu dem Lecithin/Öl-Gemisch gegeben. Diese Mischung wurde bei 35°C gerührt und nach einer Reaktionszeit von 15h unter einem Vakuum von 0,1 bar und bei einer Temperatur von 60°C getrocknet.
Ergebnis: Das erhaltene Produkt enthielt 6 Gew.-% Acetonunlösliches, was einem Lecithinanteil von 9,5% entspricht, und ferner 0,2 Gew.-% Lysophosphatidylcholin, 4 Gew.-% Monoacylglyceride und 10 Gew.-% Diacylglyceride. Das Verhältnis Lecithin zu Mono-/Diacylglyceride betrug 1 : 1,5.

### Beispiel 4

30g Rohlecithin wurden mit 270g Sojaöl gemischt und bei einer Temperatur von 60°C gerührt. 0,3g Lipase aus *Candida cylindracea* wurden in 18g Wasser gelöst und anschließend unter Rühren zu dem Lecithin/Öl-Gemisch gegeben. Diese Mischung wurde bei 60°C gerührt und nach einer Reaktionszeit von 15h unter einem Vakuum von 0,1 bar und bei einer Temperatur von 80°C getrocknet.
Ergebnis: Das erhaltene Produkt enthielt 6 Gew.-% Acetonunlösliches, was einem Lecithinanteil von 9,5% entspricht, und ferner 0,2 Gew.-% Lysophosphatidylcholin, 2 Gew.-% Monoacylglyceride und 12 Gew.-% Diacylglyceride. Das Verhältnis Lecithin zu Mono-/Diacylglyceride betrug 1 : 1,5.

### Beispiel 5

70g Rohlecithin wurde mit 30g Sojaöl gemischt und bei einer Temperatur von 35°C gerührt. 0,1g Lipase aus *Candida cylindracea* wurden in 12g Wasser gelöst und anschließend unter Rühren zu dem Lecithin/Öl-Gemisch gegeben. Diese Mischung wurde bei 35°C gerührt und nach einer Reaktionszeit von 2h unter einem Vakuum von 0,1 bar und bei einer Temperatur von 60°C getrocknet.
Ergebnis: Das erhaltene Produkt wies einen Anteil Acetonunlösliches von ca. 45 Gew.-% auf und es enthielt 3 Gew.-% Lysophosphatidylcholin, 6,5 Gew.-% Monoacylglyceride und 9 Gew.-% Diacylglyceride.

### Beispiel 6

75g Rohlecithin wurden mit 25g Sojaöl gemischt und bei einer Temperatur von 47,5°C gerührt. 0,05g Lipase aus *Candida cylindracea* wurden in 9g Wasser gelöst und anschließend unter Rühren zu dem Lecithin/Öl-Gemisch gegeben. Diese Mischung wurde bei 47,5°C über einen Zeitraum von 8,5h gerührt und anschließend unter einem Vakuum von 0,1 bar und bei einer Temperatur von 80°C getrocknet.
Ergebnis: Das erhaltene Produkt wies einen Anteil Acetonunlösliches von ca. 50 Gew.-% auf und es enthielt 4 Gew.-% Lysophosphatidylcholin, 5 Gew.-% Monoacylglyceride und 6 Gew.-% Diacylglyceride.

### Beispiel 7

225g Rohlecithin wurden mit 275g Sojaöl gemischt und bei einer Temperatur von 47,5°C gerührt. 0,25g Lipase aus *Candida cylindracea* wurden in 70g Wasser gelöst und anschließend unter Rühren zu dem Lecithin/Öl-Gemisch gegeben. Diese Mischung wurde bei 47,5°C gerührt und nach einer Reaktionszeit von 8,5h unter einem Vakuum von 0,1 bar und bei einer Temperatur von 60°C getrocknet.
Ergebnis: Das erhaltene Produkt wies einen Anteil Acetonunlösliches von ca. 30 Gew.-% auf und es enthielt 2 Gew.-% Lysophosphatidylcholin, 6 Gew.-% Monoacylglyceride und 8,5 Gew.-% Diacylglyceride.

### Beispiel 8

70g Rohlecithin wurden mit 30g Sojaöl gemischt und bei einer Temperatur von 60°C gerührt. 0,1g Lipase aus *Aspergillus niger* wurden in 12g Wasser gelöst und anschließend unter Rühren zu dem Lecithin/Öl-Gemisch gegeben. Diese Mischung wurde bei 60°C gerührt und nach einer Reaktionszeit von 15h unter einem Vakuum von 0,1 bar und bei einer Temperatur von 60°C getrocknet.
Ergebnis: Das erhaltene Produkt wies einen Anteil Acetonunlösliches von ca. 45 Gew.-% auf und es enthielt 3 Gew.-% Lysophosphatidylcholin, 5 Gew.-% Monoacylglyceride und 10 Gew.-% Diacylglyceride.

### Beispiel 9

70g Rohlecithin wurden mit 30g gehärtetem Palmfett gemischt und bei einer Temperatur von 45°C gerührt. 0,05g Lipase aus *Candida cylindracea* wurden in 10g Wasser gelöst und anschließend unter Rühren zu dem Lecithin/Öl-Gemisch gegeben. Diese Mischung wurde bei 45°C gerührt und nach einer Reaktionszeit von 8h unter einem Vakuum von 0,1 bar und bei einer Temperatur von 60°C getrocknet. Anschließend wurde das Produkt abgekühlt und auskristallisiert.
Ergebnis: Der Emulgator enthielt einen Anteil Acetonunlösliches von ca. 45 Gew.-% und ferner 3 Gew.-% Lysophosphatidylcholin, 5 Gew.-% Monoacylglyceride und 10 Gew.-% Diacylglyceride.

### Beispiel 10 (Anwendungsbeispiel)

Der nach Beispiel 9 hergestellte Emulgator wurde in einem Standardverfahren für die Herstellung von Margarine nach folgender Rezeptur eingesetzt:

| | |
|---|---|
| 47,5 Gew.-% | Sojaöl |
| 31,5 Gew.-% | Palmöl |
| 19,8 Gew.-% | Wasser |
| 0,1 Gew.-% | Kochsalz |
| 0,07 Gew.-% | Molkenprotein |
| 0,03 Gew.-% | Citronensäure |
| 1,0 Gew.-% | Emulgator |

Ergebnis: Es wurde eine Margarine erhalten, die in Bezug auf Textur, Schmelzverhalten und Streichfähigkeit Standardmargarinen gleichzusetzen ist.

## Patentansprüche

1. Verfahren zur enzymatischen Herstellung von Mono- und Diacylglycerid-haltigen Emulgatoren, **dadurch gekennzeichnet, dass**
a) eine Mischung aus einer Phospholipid- und einer Triacylglycerid-Komponente vorgelegt wird,
b) zu der Mischung aus Verfahrensschritt a) eine solche Menge einer (Phospho-)Lipase-haltigen wässrigen Lösung gegeben wird, dass der Wassergehalt der Mischung zwischen 3 und 15 Gew.-% beträgt, anschließend
c) die aus Verfahrensschritt b) erhaltene Mischung bei Temperaturen zwischen 20° und 80°C über einen Zeitraum von mindestens 2 Stunden zur Reaktion gebracht wird, und abschließend
d) die Mischung nach dem Reaktionsende getrocknet wird.

2. Verfahren nach Anspruch 1, **dadurch gekennzeichnet, dass** als Phospholipid-Komponente ein Lecithin, vorzugsweise Rohlecithin und besonders bevorzugt ein Soja-Rohlecithin, verwendet wird.

3. Verfahren nach einem der Ansprüche 1 oder 2, **dadurch gekennzeichnet, dass** als Triacylglycerid-Komponente ein pflanzliches und/oder tierisches Öl, vorzugsweise in raffinierter und/oder mind. teilweise gehärteter Form, eingesetzt wird.

4. Verfahren nach einem der Ansprüche 1 bis 3, **dadurch gekennzeichnet, dass** im Verfahrensschritt a) eine Mischung mit einem PhospholipidKomponenten-Anteil zwischen 10 und 80 Gew.-% vorgelegt wird.

5. Verfahren nach einem der Ansprüche 1 bis 4, **dadurch gekennzeichnet, dass** im Verfahrensschritt a) eine Mischung mit einem TriacylglyceridKomponenten-Anteil zwischen 20 und 90 Gew.-% vorgelegt wird.

6. Verfahren nach einem der Ansprüche 1 bis 5, **dadurch gekennzeichnet, dass** die Mischung im Verfahrensschritt a) auf eine Temperatur zwischen 35° und 60°C gebracht wird.

7. Verfahren nach einem der Ansprüche 1 bis 6, **dadurch gekennzeichnet, dass** im Verfahrensschritt b) eine Lipase- und/oder Phospholipase mikrobiellen Ursprungs, vorzugsweise aus Candida und/oder Aspergillus eingesetzt wird.

8. Verfahren nach einem der Ansprüche 1 bis 7, **dadurch gekennzeichnet, dass** eine (Phospho-)Lipasemenge von 0,05 bis 10 mg/ml verwendet wird.

9. Verfahren nach einem der Ansprüche 1 bis 8, **dadurch gekennzeichnet, dass** im Verfahrensschritt c) eine Temperatur zwischen 40° und 50°C eingestellt wird.

10. Verfahren nach einem der Ansprüche 1 bis 9, daduch **gekennzeichnet**, dass die Reaktionsdauer im Verfahrensschritt c) zwischen 5 und 20 Stunden beträgt.

11. Verfahren nach einem der Ansprüche 1 bis 10, daduch **gekennzeichnet**, dass der Trocknungsschritt d) bei Temperaturen zwischen 60 und 80 °C durchgeführt wird.

12. Verfahren nach einem der Ansprüche 1 bis 11, **dadurch gekennzeichnet, dass** ein Mischung aus Lysolecithin, Mono- und Diacylglyceriden in bevorzugten Anteilen zwischen 3,0 und 75 Gew.-% Lysolecithin, 2,0 bis 20 Gew.-% Monoacylglyceriden und 6,0 bis 40 Gew.-% Diacylglyceriden erhalten wird.

13. Verfahren nach einem der Ansprüche 1 bis 11, **dadurch gekennzeichnet, dass** ein Gemisch mit einem Verhältnis Phospholipid-Komponente : Mono- und Diacylglycerid-Komponente von 1 : 0,25 bis 4,0 erhalten wird.

14. Verfahren zur Herstellung von Emulsionen und Cremes im Lebensmittelbereich, insbesondere in Form von Eiscremes, Margarinen und Backwaren und im Kosmetikbereich, umfassend
i) die enzymatische Herstellung von Mono- und Diacylglycerid-haltigen Emulgatoren nach einem der Ansprüche 1 bis 13 und
ii) Herstellung von Emulsionen und Cremes im Lebensmittelbereich, insbesondere in Form von Eiscremes, Margarinen und Backwaren, und im Kosmetikbereich unter Einsatz der in i) erhaltenen Emulgatoren.

## Claims

1. Process for the enzymatic production of emulsifiers containing monoacylglycerides and diacylglycerides, **characterized in that**
a) a mixture of a phospholipid component and a triacylglyceride component is added first,
b) an aqueous solution containing (phospho)lipase is added to the mixture from process step a) in such an amount that the water content of the mixture is between 3 and 15 % by weight, subsequently
c) the mixture obtained from process step b) is reacted at temperatures between 20°C and 80°C for a period of at least 2 hours, and finally
d) the mixture is dried after the end of the reaction.

2. Process according to claim 1, **characterized in that** a lecithin, preferably crude lecithin and particularly preferably a crude soy lecithin is used as the phospholipid component.

3. Process according to one of the claims 1 or 2, **characterized in that** a vegetable and/or animal oil preferably in refined form and/or at least partially hardened form is used as the triacylglyceride component.

4. Process according to one of the claims 1 to 3, **characterized in that** in process step a) a mixture having a proportion of phospholipid component between 10 and 80 % by weight is added first.

5. Process according to one of the claims 1 to 4, **characterized in that** in process step a) a mixture having a proportion of triacylglyceride component between 20 and 90 % by weight is added first.

6. Process according to one of the claims 1 to 5, **characterized in that** the mixture in process step a) is brought to a temperature between 35°C and 60° C.

7. Process according to one of the claims 1. to 6, **characterized in that** in process step b) a lipase and/or phospholipase of microbial origin preferably from Candida and/or Aspergillus is used.

8. Process according to one of the claims 1 to 7, **characterized in that** a (phospho)lipase amount of 0.05 to 10 mg/ml is used.

9. Process according to one of the claims 1 to 8, **characterized in that** a temperature between 40°C and 50°C is set in process step c).

10. Process according to one of the claims 1 to 9, **characterized in that** the reaction period in process step c) is between 5 and 20 hours.

11. Process according to one of the claims 1 to 10, **characterized in that** the drying step d) is carried out at temperatures between 60 and 80°C.

12. Process according to one of the claims 1 to 11, **characterized in that** a mixture is obtained of lysolecithin, monoacylglycerides and diacylglycerides in preferred proportions of between 3.0 and 75 % by weight lysolecithin, 2.0 to 20 % by weight monoacylglycerides and 6.0 to 40 % by weight diacylglycerides.

13. Process according to one of the claims 1 to 11, **characterized in that** a mixture is obtained having a ratio of phospholipid component: monoacylglyceride and diacylglyceride component of 1 : 0.25 to 4.0.

14. Process for producing emulsions and creams in the food sector, in particular in the form of ice creams, margarines and bakery products and in the cosmetic sector, comprising
i) the enzymatic production of emulsifiers containing monoglycerides and diacylglycerides according to one of the claims 1 to 13 and
ii) production of emulsions and creams in the food sector, in particular in the form of ice creams, margarines and bakery products and in the cosmetic sector using the emulsifiers obtained in i).

## Revendications

1. Procédé de production enzymatique d'émulsifiants contenant des mono- et diacylglycérides, **caractérisé en ce que**
a) un mélange d'un composant phospholipide et d'un composant triacylglycéride est préparé,
b) on ajoute au mélange de l'étape a) du procédé, une quantité d'une solution aqueuse contenant une (phospho)-lipase en une quantité telle que la teneur en eau du mélange se situe entre 3 et 15 % en poids, ensuite
c) on fait réagir le mélange obtenu à l'étape b) du procédé à des températures entre 20° et 80° C sur une durée d'au moins 2 heures, et ensuite
d) le mélange est séché après la fin de la réaction.

2. Procédé selon la revendication 1, **caractérisé en ce que** l'on utilise comme composant phospholipide une lécithine, de préférence une lécithine brute et de manière particulièrement préférée une lécithine brute de soja.

3. Procédé selon l'une des revendications 1 ou 2, **caractérisé en ce que** l'on utilise comme composant triacylglycéride une huile végétale et/ou une huile animale, de préférence sous forme raffinée et/ou sous forme au moins partiellement durcie.

4. Procédé selon l'une des revendications 1 à 3, **caractérisé en ce que**, à l'étape a) du procédé est préparé un mélange présentant une teneur en composant phospholipide entre 10 et 80 % en poids.

5. Procédé selon l'une des revendications 1 à 4, **caractérisé en ce que**, à l'étape a) du procédé est préparé un mélange présentant une teneur en composant triacylglycéride entre 20 et 90 % en poids.

6. Procédé selon l'une quelconque des revendications 1 à 5, **caractérisé en ce que** le mélange à l'étape a) du procédé est porté à une température entre 35° et 60° C.

7. Procédé selon l'une des revendications 1 à 6, **caractérisé en ce que** l'on utilise à l'étape b) du procédé, une lipase et/ou une phospholipase d'origine microbienne, de préférence issue de *Candida* et/ou d'*Aspergillus.*

8. Procédé selon l'une des revendications 1 à 7, **caractérisé en ce que** l'on utilise une quantité de (phospho)lipase de 0,05 à 10 mg/ml.

9. Procédé selon l'une des revendications 1 à 8, **caractérisé en ce qu'**à l'étape c) du procédé, la température est réglée entre 40° et 50° C.

10. Procédé selon l'une des revendications 1 à 9, **caractérisé en ce que** la durée de réaction à l'étape c) du procédé se situe entre 5 et 20 heures.

11. Procédé selon l'une des revendications 1 à 10, **caractérisé en ce que** l'étape d) de séchage est réalisée à des températures entre 60 et 80° C.

12. Procédé selon l'une des revendications 1 à 11, **caractérisé en ce que** l'on obtient un mélange de lysolécithine, de mono- et diacylglycérides en proportions de préférence entre 3,0 et 75 % en poids de lysolécithine, de 2,0 à 20 % en poids de monoacylglycérides et de 6,0 à 40 % en poids de diacylglycérides.

13. Procédé selon l'une des revendications 1 à 11, **caractérisé en ce que** l'on obtient un mélange en un rapport composant phospholipide : composant mono- et diacylglycéride de 1 : 0,25 à 4,0.

14. Procédé de production d'émulsions et de crèmes dans le domaine alimentaire, en particulier sous la forme de crèmes glacées, de margarines et de pâtisseries et dans le domaine cosmétique, comprenant
i) la production enzymatique d'émulsifiants contenant des mono- et diacylglycérides selon l'une des revendication 1 à 13 et
ii) la production d'émulsions et de crèmes dans le domaine alimentaire, en particulier sous la forme de crèmes glacées, de margarines et de pâtisseries et dans le domaine cosmétique en utilisant les émulsifiants obtenus au point i).
